## Europäisches Patentamt
## European Patent Office
## Office européen des brevets

(19)

(11) Numéro de publication : **0 013 528**
**B1**

(12)

# FASCICULE DE BREVET EUROPÉEN

(45) Date de publication du fascicule du brevet :
01.09.82

(51) Int. Cl.³ : **G 01 N 33/18**

(21) Numéro de dépôt : 79401072.8

(22) Date de dépôt : 27.12.79

(54) Procédé de détermination de la concentration des bases libres dans une eau industrielle et dispositif de mesure pour sa mise en oeuvre.

(30) Priorité : 29.12.78 FR 7837016

(43) Date de publication de la demande :
23.07.80 (Bulletin 80/15)

(45) Mention de la délivrance du brevet :
01.09.82 Bulletin 82/35

(84) Etats contractants désignés :
BE CH DE GB IT NL SE

(56) Documents cités :
DE A 1 573 978
FR A 2 288 310
US A 2 832 673
US A 3 915 642
SOVIET JOURNAL OF INSTRUMENTATION AND
CONTROL, no. 10, octobre 1968, V.I.E. LISEEV :
« Automatic analysis of steam condensate »,
pages 64-65.

(73) Titulaire : Framatome
Tour Flat 1 place de la Coupole
F-92400 Courbevoie (FR)

(72) Inventeur : Saurin, Pierre
9 Le Manoir de Denouval
F-78570 Andresy (FR)
Inventeur : Nordmann, Francis
7 Mail des Catalpas
F-78390 Bois d'Arcy (FR)

(74) Mandataire : Bouget, Lucien et al
CREUSOT-LOIRE 15 rue Pasquier
F-75383 Paris Cedex 08 (FR)

EP 0 013 528 B1

Imprimerie Jouve, 18, rue St-Denis, 75001 Paris, France

# Procédé de détermination de la concentration des bases libres dans une eau industrielle et dispositif de mesure pour sa mise en œuvre

L'invention concerne un procédé de détermination de la concentration des bases libres dans une eau industrielle subissant un ensemble de traitements susceptibles de faire varier sa concentration en polluants sous forme de bases et de sels et un dispositif de mesure pour sa mise en œuvre.

Dans le cas où l'on utilise de l'eau en grande quantité par exemple pour le refroidissement d'installations de production d'énergie ou pour la production de vapeur, dans un but industriel, il est nécessaire d'opérer sur cette eau un certain nombre de traitements qui peuvent être inhérents au processus de l'utilisation de l'eau ou qui peuvent être nécessaires pour modifier ses caractéristiques chimiques tels que sa composition ou son pH.

Dans le cas des réacteurs nucléaires à eau sous pression, on utilise une circulation d'eau à haute pression en circuit fermé pour transporter la chaleur produite dans le réacteur et transférer cette chaleur à l'intérieur de générateurs de vapeur, à de l'eau alimentaire qui se transforme en vapeur au contact de tubes à l'intérieur desquels circule l'eau transportant la chaleur du réacteur ou eau primaire.

La vapeur produite est envoyée à la turbine de production d'énergie condensée en sortie de la turbine et recyclée après passage sur des réchauffeurs. La circulation de l'eau secondaire entre le générateur de vapeur, la turbine, le condenseur et les réchauffeurs n'est cependant pas tout à fait une circulation en circuit fermé puisque, d'une part on réalise la purge d'une certaine partie de l'eau secondaire au niveau du générateur de vapeur et que d'autre part on ajoute une certaine quantité d'eau d'appoint traitée préalablement à son introduction dans le circuit secondaire du réacteur.

Afin d'éviter des problèmes de corrosion, d'entartrement ou d'encrassement du circuit alimentaire, il est nécessaire d'une part d'effectuer certains traitements de l'eau secondaire et d'autre part de contrôler la teneur de cette eau en divers éléments polluants, en particulier en éléments polluants sous forme de sels ou de bases.

En effet, de tels polluants peuvent être introduits avec l'eau d'appoint, en cas de mauvais fonctionnement de l'installation de traitement de cette eau d'appoint ou par introduction d'eau brute de refroidissement au niveau du condenseur, dans le cas où ce condenseur présente des défauts d'étanchéité.

L'eau de refroidissement du condenseur ou eau brute, peut être suivant l'emplacement de la centrale nucléaire, de l'eau de rivière ou de l'eau de mer.

En particulier, des bases telles que la soude ou des hydroxydes de métaux alcalino-terreux peuvent être introduites dans l'eau secondaire et ceci peut provoquer l'apparition d'une corrosion caustique (ou inter-granulaire) au niveau des éléments du générateur de vapeur.

Il est donc extrêmement important de mesurer avec précision la concentration en bases ou hydroxydes de l'eau des purges des générateurs de vapeur et/ou de l'eau alimentaire qu'on appellera eau secondaire dans les deux cas, pour éviter que cette concentration ne devienne excessive. Lorsqu'un écart par rapport aux limites prévues pour ces concentrations est détecté, il est alors possible d'agir sur les unités de traitement de l'eau ou d'interrompre le fonctionnement d'une unité de traitement défectueuse.

D'autre part, on ajoute généralement à l'eau alimentaire de l'ammoniaque et de l'hydrazine pour son conditionnement, l'ammoniaque assurant l'ajustement du Ph de l'eau et l'hydrazine permettant de régler la concentration en oxygène de l'eau alimentaire. On injecte donc certaines quantités d'ammoniaque et d'hydrazine en un point du circuit secondaire.

On appellera bases libres toute base ou hydroxyde présentes dans l'eau secondaire c'est-à-dire apportant dans cette eau secondaire des ions $OH^-$, à l'exception des bases introduites pour le conditionnement de l'eau alimentaire.

C'est évidemment la concentration en bases libres qui détermine l'agressivité de l'eau secondaire en ce qui concerne la corrosion caustique ou intergranulaire.

Jusqu'ici, le contrôle de la concentration en bases libres était fait à partir d'une mesure de la concentration totale en base de l'eau par potentiométrie, la valeur de la concentration en bases libres se déduisant de cette valeur de mesure en en retranchant la concentration en ammoniaque et en hydrazine obtenue par un dosage avec le réactif de Nessler et avec la paradiméthylamino-benzaldéhyde.

Cette méthode de détermination présente des inconvénients car la précision des mesures est extrêmement faible et le dosage de l'ammoniaque et de l'hydrazine ne peuvent pas être faits en continu.

En dehors de la détermination de la concentration en bases libres de l'eau secondaire, on effectue d'autres contrôles et mesures sur cette eau permettant d'évaluer sa conformité par rapport aux normes requises et en particulier de contrôler son inocuité par rapport aux matériaux avec lesquels elle vient en contact.

Parmi ces méthodes de mesure, on peut citer celle décrite dans la revue « Soviet journal of instrumentation and control », n° 10, d'octobre 1968, dans laquelle on détermine la concentration en NaCl par différence entre la conductivité totale de la solution et celle due à la soude.

On peut citer aussi le brevet US 2.832.673 qui décrit une mesure de concentration en sels par mesure de la conductivité de la solution après son passage sur une résine échangeuse d'ions, remplaçant les cations par des ions $H^+$.

On peut citer enfin une mesure de pH dont la valeur peut être ajustée grâce à l'ammoniaque.

On effectue également, de façon assez courante, une mesure de la concentration totale en cations par une méthode chimique ou électrochimique telle qu'une mesure de potentiel avec une électrode spécifique de chacun des cations à doser.

Toutes ces mesures ne fournissent cependant pas d'éléments en ce qui concerne la concentration en bases libres pour laquelle on ne dispose que d'une méthode de détermination peu précise.

Le but de l'invention est donc de proposer un procédé de détermination de la concentration en bases libres dans une eau industrielle subissant un ensemble de traitements susceptibles de faire varier la concentration en polluants sous forme de bases et de sels, comprenant une mesure de la conductivité de l'eau, après un passage de cette eau sur des résines échangeuses d'ions retenant les cations en solution et libérant à la place des ions $H^+$, et une détermination de la valeur de la concentration en cations venant des sels à partir de cette mesure par utilisation de la relation linéaire existant entre la conductivité cationique et la concentration en cations provenant des sels, caractérisé en ce qu'on effectue d'autre part une mesure de la concentration totale en cations provenant des sels et des bases par association des mesures faites à partir d'électrodes connues en elles-mêmes spécifiques pour chaque type de cations susceptibles d'être présents, et en ce qu'on calcule la différence entre la concentration totale en cations et la concentration en cations provenant des sels, le résultat de cette différence donnant la valeur de la concentration en bases libres de l'eau.

Afin de bien faire comprendre l'invention, on va maintenant décrire à titre d'exemple non limitatif un mode de réalisation du procédé suivant l'invention appliqué à l'eau secondaire d'une centrale nucléaire à eau sous pression, dans le cas où l'eau brute de refroidissement du condenseur est de l'eau de rivière et dans le cas où cette eau est de l'eau de mer.

La figure 1 représente de façon schématique le circuit secondaire du réacteur nucléaire.

La figure 2 représente de façon schématique, le dispositif de mesure de la concentration des bases libres dans l'eau secondaire du réacteur.

La figure 3 est un graphique montrant les variations de la concentration en cations par rapport à la conductivité cationique, dans le cas d'une eau polluée par une eau de rivière.

La figure 4 est un graphique montrant les variations de la concentration en cations d'une eau polluée par de l'eau de mer, en fonction de la conductivité cationique, cette représentation utilisant une échelle logarithmique.

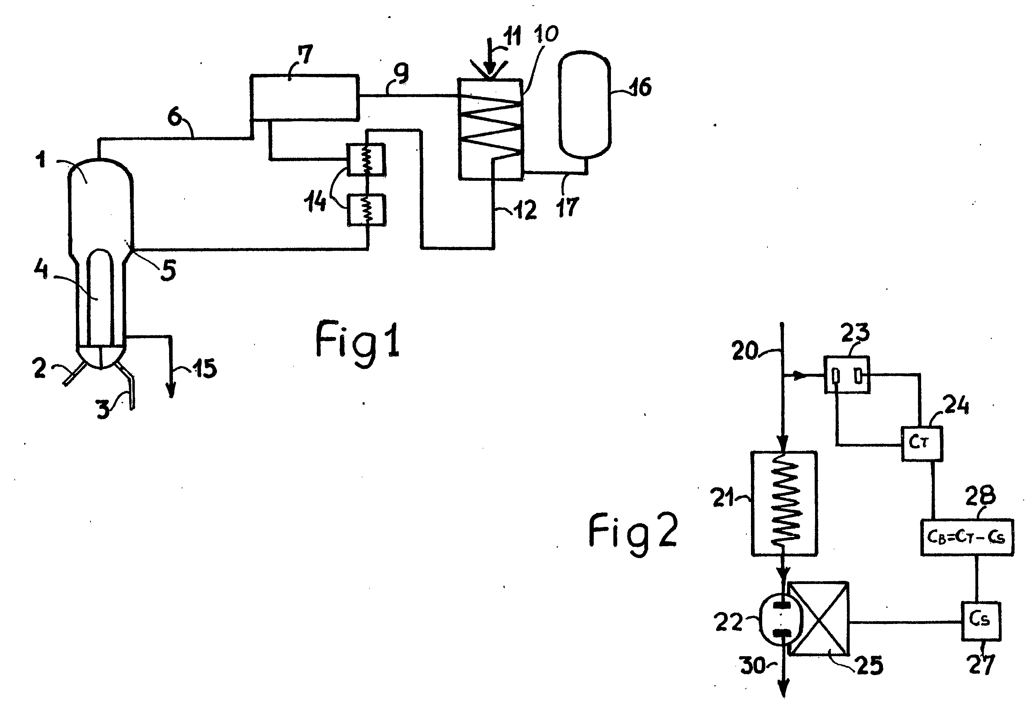

Sur la figure 1, on voit un circuit secondaire d'un réacteur nucléaire dont le générateur de vapeur 1 est alimenté à sa base en eau primaire par des portions 2 et 3 du circuit primaire, cette eau primaire circulant dans le générateur de vapeur à l'intérieur du faisceau tubulaire 4.

L'eau alimentaire est amenée en 5 dans le générateur de vapeur alors que la vapeur est soutirée à la partie supérieure du générateur de vapeur par une canalisation 6.

La vapeur est alors amenée à la turbine 7 à l'intérieur de laquelle elle fournit la puissance motrice et ressort de la turbine par une conduite 9 l'amenant au condenseur 10.

A l'intérieur du condenseur, la vapeur est refroidie par une circulation d'eau brute introduite en 11 dans le condenseur, si bien que l'eau condensée est envoyée par la conduite 12 aux réchauffeurs 14 où l'eau condensée est réchauffée par de la vapeur soutirée au niveau de la turbine.

L'eau secondaire réchauffée est alors réintroduite en 5 dans le générateur de vapeur.

A la base du générateur de vapeur, au niveau de la plaque tubulaire, on a prévu une purge 15 permettant d'éliminer une partie de la vapeur et de l'eau se trouvant dans le générateur de vapeur, par exemple pour l'élimination d'impuretés par filtration ou par épuration sur des résines échangeuses d'ions.

Au niveau du condenseur, on a prévu également un réservoir d'eau d'appoint 16 en communication avec le circuit du condenseur par une canalisation 17 permettant d'introduire dans le circuit secondaire des appoints d'eau qui peuvent être réchauffés avec les condensats dans les réchauffeurs 14 avant leur introduction dans le générateur de vapeur en 5.

L'eau du réservoir d'appoint est une eau déminéralisée par un traitement d'échange d'ions et qui n'introduit donc dans le circuit secondaire des produits polluants en concentration notable qu'en cas de mauvais fonctionnement de l'installation de déminéralisation.

Le contrôle de la concentration de bases libres dans l'eau du circuit secondaire se fait généralement au niveau des purges 15 du générateur de vapeur.

Cependant il est possible également d'effectuer cette mesure de concentration sur des condensats recyclés en sortie du condenseur 10 ou sur l'eau du réservoir d'appoint. On peut également vérifier la concentration des bases libres dans l'eau alimentaire au moment de l'introduction de l'eau alimentaire dans les réchauffeurs.

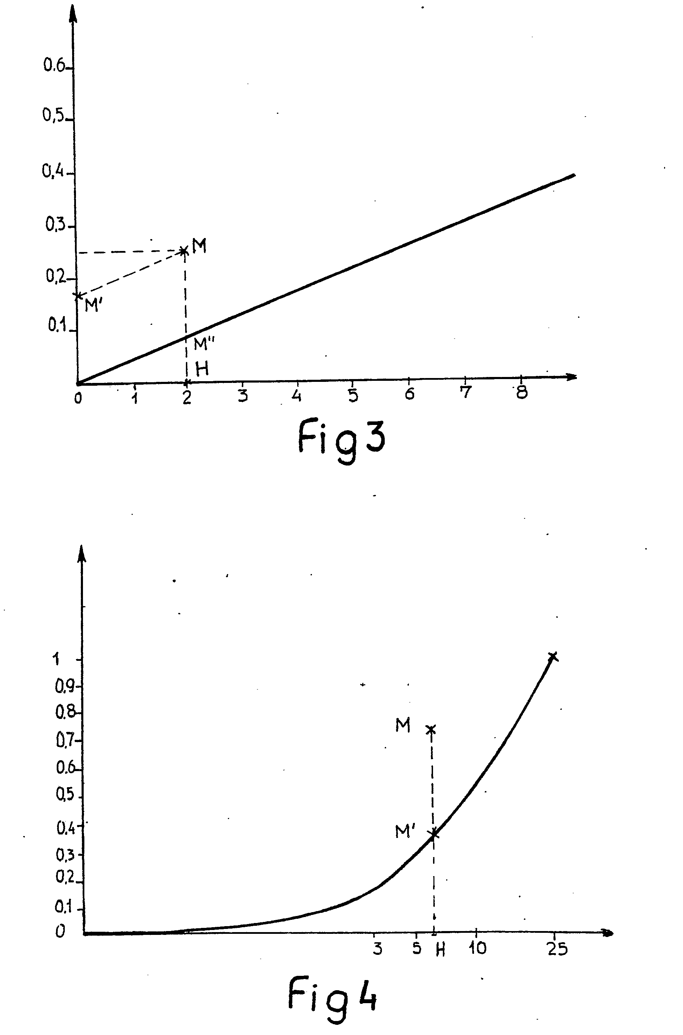

De toute façon, l'ensemble de l'installation de mesures de la concentration de bases libres est du type représenté à la figure 2.

Sur la figure 2 on voit une canalisation d'arrivée d'eau 20, cette eau provenant par exemple des purges du générateur de vapeur.

Une partie de cette eau arrivant par la canalisation 20 est dérivée dans un dispositif de mesure potentiométrique 23 comportant une électrode spécifique à chacun des cations à analyser, ce dispositif 23 étant associé à un module 24 fournissant en sortie un signal proportionnel à la concentration totale en cations $C^T$.

L'eau à analyser arrivant par la canalisation 20 est introduite dans une unité de déminéralisation à résine échangeuse d'ions 21 ou colonne échangeuse d'ions, dans laquelle les cations en solution sont remplacés par des ions $H^+$.

L'eau au sortir de l'unité de déminéralisation à échange d'ions pénètre dans une cellule de mesure de conductivité 22 formant l'une des branches d'un pont de mesure 25 permettant grâce à un module de mesure 27 qui lui est associé, la sortie d'un signal proportionnel à la concentration en cations provenant des sels en solution dans l'eau $C^S$.

Les signaux proportionnels à $C^T$ et $C^S$ sont introduits dans un sommateur 28 qui permet d'engendrer un signal proportionnel à la différence $C^B$ entre $C^T$ et $C^S$.

Le signal $C^B$ dont nous verrons qu'il représente la concentration en bases libres dans l'eau peut être utilisé pour toute opération d'affichage, de commande ou de déclenchement d'alarme qui peuvent être utiles pour la conduite des unités de traitement de l'eau du circuit secondaire du réacteur nucléaire.

On va maintenant décrire, en se référant aux figures 3 et 4, la façon dont est réalisée la détermination de la concentration en bases libres de l'eau, dans le cas où cette eau est polluée par de l'eau de rivière et dans le cas où cette eau est polluée par de l'eau de mer.

Dans le cas où l'eau est polluée par l'eau de rivière, c'est-à-dire dans le cas où cette eau a pu être introduite au niveau du condensateur si ce dernier a des défauts d'étanchéité, les ions polluants sont pratiquement des ions sodium (Na) et calcium (Ca), à l'exception d'autres cations en faible proportion tels que le magnésium (Mg) et le potassium (K).

Généralement le rapport Na + Ca/Na + Ca + Mg + K est voisin de l'unité et sensiblement égal à 0,9. Les cations en solution peuvent provenir aussi bien de sels tels que NaCl, CaCl₂... que de bases ou hydroxydes tels que la soude NaOH, Ca (OH)₂, Mg (OH)₂ ou KOH.

Ces hydroxydes sont issus de la décomposition thermique des bicarbonates présents dans l'eau de rivière.

L'ensemble des cations qui proviennent des sels ou des bases sont dosés au niveau du dispositif de mesure potentiométrique 23 qui fournit grâce au module de mesure 24 un signal proportionnel à la concentration totale en cations dans l'eau dont on effectue l'analyse.

Au moment du passage de l'eau dans l'unité de déminéralisation à résine échangeuse d'ions, les cations sont retenus par la résine qui libère à leur place des ions H⁺ qui entrent en solution, cependant que les anions restent en solution.

Dans le cas d'un sel, par exemple NaCl, on libère ainsi un ion H⁺ et un ion Cl⁻, ce qui a une influence sur la conductivité de la solution qui devient une solution d'acide chlorhydrique.

Par contre dans le cas d'une base, par exemple NaOH, on libère un ion OH⁻ qui se recombine avec l'ion H⁺ pour donner de l'eau, si bien que globalement cet échange d'ions annule l'effet des bases sur la conductivité de la solution.

La mesure de la conductivité au niveau de la cellule de mesure 22, cette conductivité étant appelée conductivité cationique, donne donc des résultats qui sont uniquement proportionnels à la concentration en cations venant des sels dans la solution, puisque les cations venant des bases ont été éliminés avec production d'eau.

L'eau prélevée au niveau des purges des générateurs de vapeur contient également de l'ammoniaque NH₄OH et de l'hydrazine NH₂NH₂. Une résine du type cationique acide fort échangeuse d'ions permet de retenir l'ammoniaque et l'hydrazine en solution dans l'eau au niveau de l'unité de déminéralisation.

Sur la figure 3, on a représenté les variations de la concentration en cations sodium plus calcium dans une eau polluée par une eau de rivière cette concentration étant exprimée en mg par kilogramme de solution, en fonction de la conductivité cationique de la solution, c'est-à-dire la conductivité telle que mesurée dans la cellule 22 après passage de l'eau dans l'unité de traitement 21.

On voit que la relation entre concentration et conductivité est une relation linéaire et qu'ainsi à chacune des valeurs de la conductivité correspond une valeur de la concentration en ions sodium et calcium venant des sels qui lui est proportionnelle.

Par exemple dans le cas d'une eau de conductivité cationique égale à 2 µS/cm (point H sur l'axe des abscisses), correspond une concentration en sodium plus calcium venant des sels égale à HM″, M″ étant le point correspondant sur la droite représentative de la relation linéaire entre conductivité et concentration.

Si le point M représente sur le même graphique la concentration totale en ions sodium plus calcium de l'eau telle que mesurée dans le dispositif de mesure potentiométrique 23, exprimée en mg/Kg, la longueur HM représente la concentration totale en ions sodium plus calcium $C^T$.

Comme l'ensemble des ions sodium et calcium dans l'eau proviennent soit des sels en solution soit des bases à l'exclusion des bases ajoutées pour le conditionnement de l'eau et retenues au niveau de l'unité de traitement 21, on obtient la concentration en bases libres de l'eau par simple différence entre $C^T$ et $C^S$, c'est à dire entre HM et HM″.

Ainsi la concentration en bases libres $C^B$ peut être représentée sur le graphique de la figure 3 par longueur MM″ ou encore par longueur OM′, MM′ étant parallèle à la droite représentative de la relation linéaire entre conductivité et concentration.

On voit donc que la connaissance de la relation linéaire entre conductivité et concentration en cations polluants, qui peut être connu à partir du moment où l'on connaît ses cations, permet une résolution graphique simple du problème de la détermination de la concentration en bases libres à partir de la connaissance de la conductivité cationique fournie par la mesure au niveau du pont de mesure 25 et de la concentration totale en cations fournie au niveau du dispositif de mesure potentiométrique 23.

On peut donc substituer à un traitement de signaux représentatifs des concentrations, une méthode de résolution graphique permettant d'obtenir très simplement la valeur de la concentration des bases libres dans l'eau.

On a représenté sur la figure 4, en utilisant des coordonnées logarithmiques pour la conductivité cationique qui est susceptible de varier dans des proportions plus importantes pour des variations usuelles de la concentration en polluants de l'eau, la courbe représentative des variations de cette concentration en fonction de la conductivité.

Dans le cas d'une pollution de l'eau secondaire par l'eau de mer, le cation polluant prépondérant et pratiquement unique sera le sodium, sous forme de chlorure.

Dans ce cas, de la soude présente dans l'eau secondaire peut provenir essentiellement de l'eau d'appoint, lorsque l'unité de traitement de l'eau d'appoint fonctionne dans de mauvaises conditions.

Dans le cas d'un dosage réalisé sur de l'eau polluée par de l'eau de mer, en utilisant l'appareillage tel que représenté à la figure 2, on a mesuré au niveau du dispositif potentiométrique 23 une teneur totale en sodium dans l'eau de 0,74 mg/kg d'eau. On a mesuré après passage sur l'unité de traitement à résine échangeuse d'ions 21 une conductivité cationique, au niveau de la cellule de conductivité 22, de 6 µ/cm.

En se reportant à la figure 4, ces deux données, numériques permettent la détermination du point M dont on déduit la détermination du point M', la longueur MM' représentant alors la concentration en sodium sous forme de soude c'est-à-dire la teneur en bases libres de l'eau sur laquelle on effectue le dosage. Cette longueur MM' représente approximativement 0,40 mg/kg de sodium sous forme de soude dans l'eau sur laquelle on effectue le dosage.

Comme dans le cas d'une détermination d'un signal représentatif de la concentration en bases libres $C^B$, c'est-à-dire dans le cas d'un traitement analogique, on peut utiliser les valeurs de la concentration en bases libres obtenue pour surveiller le fonctionnement des unités de traitement de l'eau et du condenseur et éventuellement déclencher une alarme si la valeur de la concentration de bases libres dépasse un certain seuil prédéterminé.

On peut également déterminer des zones des diagrammes tels que ceux représentés aux figures 3 et 4 à l'intérieur desquelles doivent constamment se situer les points représentant les concentrations en cations dans l'eau.

Les signaux fournis par un dispositif du type de celui représenté à la figure 2 peuvent également être utilisés dans un dispositif plus complexe de surveillance du réacteur prenant en compte un nombre plus important de paramètres.

On voit que les principaux avantages du dispositif selon l'invention sont tout d'abord une simplification et une meilleure précision des méthodes de dosage, puisqu'on utilise une mesure de conductivité plus sensible qu'une mesure potentiométrique aux variations de composition de l'eau, pour la détermination de la concentration en cations venant des sels.

D'autre part on n'a pas effectué un dosage particulier de l'ammoniaque et de l'hydrazine contenues dans l'eau, les mesures étant toutes relatives aux cations des matières polluantes sous forme de sels ou de bases. Pour cette raison également, on peut rendre les mesures entièrement continues, puisque la mesure de conductivité au niveau de la cellule 22 peut très bien se faire en continu sur un flux d'eau en écoulement continu entre la canalisation 20 et la canalisation de sortie 30 et que de même la mesure potentiométrique au niveau du dispositif 23 peut également être faite en continu.

Le fait de disposer de mesures plus fiables et plus fréquentes, ou même de disposer de mesures en continu, permet de détecter de façon extrêmement rapide une déviation anormale de la concentration en bases libres et de prévenir ainsi l'apparition de phénomènes de corrosion caustique (ou inter-granulaire) provoquées par une élévation anormale de la concentration de la teneur en bases libres.

Le procédé de détermination et de surveillance de la concentration de bases libres dans l'eau est également très facilement automatisable car on peut obtenir une sortie ou un affichage numérique d'un paramètre permettant d'agir automatiquement sur les unités de traitement de l'eau secondaire du réacteur.

L'invention ne se limite pas aux modes de réalisations qui ont été décrits, elle en comporte au contraire toutes les variantes. C'est ainsi qu'on a décrit un appareillage de mesure comportant une cellule de conductivité disposées en série avec une unité de traitement à résine échangeuse d'ions, cette cellule de conductivité constituant l'une des branches d'un pont de mesure mais qu'il est également possible d'envisager d'autres dispositifs de mesure de conductivité cationique de la solution dont on effectue le dosage.

De la même façon on a décrit un dispositif de mesure potentiométrique de la concentration totale en cations polluants mais il est également possible d'envisager tout autre dispositif de mesure continu ou discontinu de cette concentration, ce dispositif et la méthode de mesure correspondante pouvant être adaptés au type de cations dont on veut connaître la concentration.

Enfin les résultats de mesure peuvent être traités sous forme numérique ou analogique, manuellement ou automatiquement ou encore sous forme graphique.

Au lieu d'un sommateur fournissant la différence entre les signaux venant des modules de mesure, on peut utiliser toute unité de calcul ou de traitement capable de déterminer la concentration en bases libres sous forme numérique ou analogique, à partir des mesures de concentration totale en cations et de conductivité cationique, en prenant en compte la relation linéaire entre la concentration cationique venant des sels et la conductivité cationique.

Dans le cas d'une eau très pure, il faudra cependant tenir compte de la conductivité intrinsèque de l'eau.

Le procédé suivant l'invention s'applique bien entendu aux réacteurs nucléaires et en particulier aux réacteurs nucléaires à eau sous pression, pour la surveillance de la concentration en bases libres dans l'eau secondaire, mais il s'applique également à toute autre installation industrielle utilisant de l'eau pour le refroidissement, pour la production de vapeur ou pour tout autre usage nécessitant une qualité particulière de l'eau industrielle utilisée.

**Revendications**

1. Procédé de détermination de la concentration en bases libres, c'est-à-dire en bases ou hydroxydes apportant en solution aqueuse des ions $OH^-$ à l'exception des bases introduites pour le conditionnement de l'eau de la solution, dans une eau industrielle subissant un ensemble de traitements susceptibles de faire varier la concentration en polluants sous forme de bases et de sels, comprenant une mesure de la conductivité de l'eau, après un passage de cette eau sur des résines échangeuses d'ions retenant les cations en solution et libérant à la place des ions $H^+$ et une détermination de la valeur de la concentration en cations venant des sels à partir de cette mesure par utilisation de la relation linéaire existant entre la conductivité cationique et la concentration en cations provenant des sels, caractérisé en ce qu'on effectue d'autre part une mesure de la concentration totale en cations provenant des sels et des bases par association des mesures faites à partir d'électrodes connues en elles-mêmes spécifiques pour chaque type de cations susceptibles d'être présents, et en ce que l'on calcule la différence entre la concentration totale en cations et la concentration en cations provenant des sels, le résultat de cette différence donnant la valeur de la concentration en bases libres de l'eau.

2. Procédé de détermination de la concentration suivant la revendication 1, dans le cas de l'eau secondaire d'un réacteur nucléaire à eau sous pression dont le condenseur est refroidi à l'eau de rivière, caractérisé par le fait qu'on mesure la concentration totale en sodium et en calcium dans l'eau grâce à une électrode spécifique (23), qu'on mesure la conductivité cationique de l'eau dans une cellule de conductivité (22) associée à un pont de mesure (25) et qu'on en déduit la concentration en sodium et en calcium venant des sels en solution à partir de la relation linéaire existant entre la conductivité et la concentration en sodium et en calcium.

3. Procédé de détermination de la concentration des bases libres suivant la revendication 1 dans le cas de l'eau secondaire d'un réacteur nucléaire dont le condenseur est refroidi à l'eau de mer, caractérisé par le fait qu'on mesure grâce à un dispositif de mesure potentiométrique (29) à électrode spécifique la concentration totale en ion sodium dans l'eau, qu'on mesure la conductivité cationique de cette eau, qu'on en déduit la concentration en ion sodium provenant des sels en solution à partir de la relation linéaire entre la conductivité et la concentration en sel de sodium.

4. Procédé suivant l'une quelconque des revendications 1, 2 et 3, caractérisé par le fait qu'on engendre un signal proportionnel à la concentration totale en cations polluants dans l'eau, qu'on engendre un second signal proportionnel à la concentration en cations provenant des sels dans l'eau et qu'on détermine par différence entre ces deux signaux un signal représentatif de la concentration en bases libres dans l'eau.

5. Procédé de détermination de la concentration des bases libres suivant l'une quelconque des revendications 1, 2 et 3 caractérisé par le fait qu'on trace la droite représentant les variations de la concentration en cations provenant des sels en fonction de la conductivité cationique sur un graphique, qu'on porte sur ce graphique le point ayant pour abscisse la conductivité cationique mesurée de l'eau et pour ordonnée la concentration totale en cations polluants dans l'eau et qu'on détermine la longueur du segment parallèle à l'axe des concentrations joignant le point à la droite, cette longueur suivant l'axe des concentrations donnant la concentration en bases libres.

6. Procédé de détermination de la concentration des bases libres suivant l'une quelconque des revendications 1, 2, 3, 4 et 5 caractérisé par le fait qu'on effectue les mesures de concentration totale en cations polluants et de conductivité cationique, en continu.

7. Dispositif de mesure pour la mise en œuvre du procédé selon les revendications 1, 2, 3, 4, 5 et 6, caractérisé par le fait qu'il comporte :
— une colonne échangeuse d'ions (21),
— une cellule de mesure de conductivité (22) en série avec la colonne échangeuse d'ions,
— au moins une électrode spécifique (23) de mesure de la concentration totale en cations,
— une unité de calcul (28) pour le traitement des signaux représentatifs des mesures de concentration totale en cations et de la conductivité, pour en déduire la concentration en bases libres.

**Claims**

1. A method of determination of the concentration of free bases, that is to say, of bases or hydroxides contributing $OH^-$ ions into aqueous solution, with the exception of the bases introduced for the conditioning of the water of the solution, in an industrial water undergoing a set of treatments capable of making the concentration of pollutants in the form of bases and salts vary, comprising a measurement of the conductivity of the water after passing this water over ion-exchange resins which retain the cations in the solution and free instead $H^+$ ions and a determination of the value of the concentration of cations coming from the salts beginning with this measurement by the employment of the linear relationship existing between the cationic conductivity and the concentration of cations arising from the salts, characterized in that furthermore a measurement is carried out, of the total concentration of cations arising from the salts and from the bases by association of the measurements made from electrodes known in themselves, which are specific for each type of cation capable of being present, and in that the difference is calculated between the total concentration of cations and the concentration of cations arising from the salts, the result of this difference giving the value of the concentration of free bases in the water.

2. A method of determination of concentration as in Claim 1, in the case of the secondary water in a pressurized-water nuclear reactor the condenser of which is cooled by river water, characterized by the fact that the total concentration of sodium and of calcium in the water is measured thanks to a specific electrode (23), that the cationic conductivity of the water is measured in a conductivity cell (22) associated with a measuring bridge (25) and that the concentration of sodium and of calcium coming from the salts in solution is deduced from the linear relationship existing between the conductivity and the concentration of sodium and of calcium.

3. A method of determination of the concentration of free bases as in Claim 1 in the case of the secondary water in a nuclear reactor the condenser of which is cooled by seawater, characterized by the fact that thanks to a potentiometric measuring device (29) having a specific electrode the total concentration of sodium ion in the water is measured, that the cationic conductivity of this water is measured and that from it the concentration of sodium ion arising from the salts in solution is deduced from the linear relationship between the conductivity and the concentration of sodium salt.

4. A method as in any one of the Claims 1, 2 and 3, characterized by the fact that a signal is generated which is proportional to the total concentration of pollutant cations in the water, that a second signal is generated which is proportional to the concentration of cations arising from the salts in the water and that by difference between these two signals a signal is derived which is representative of the concentration of free bases in the water.

5. A method of determination of the concentration of free bases as in any one of the Claims 1, 2 and 3, characterized by the fact that on a graph the straight line is drawn which represents the variations in the concentration of cations arising from the salts as a function of the cationic conductivity, that on this graph the point is plotted having as its abscissa the measured cationic conductivity of the water and as its ordinate the total concentration of pollutant cations in the water and that the length is determined of the segment parallel with the concentrations axis, joining the point to the straight line, this length along the concentrations axis giving the concentration of free bases.

6. A method of determination of the concentration of free bases as in any one of the Claims 1, 2, 3, 4 and 5, characterized by the fact that the measurements of total concentration of pollutant cations and of cationic conductivity are carried out continuously.

7. A measuring device for putting into effect the method as in Claims 1, 2, 3, 4, 5 and 6, characterized by the fact that it includes ;
— an ion-exchange column (21) ;
— a conductivity measuring cell (22) in series with the ion-exchange column ;
— at least one specific electrode (23) for measuring the total concentration of cations ;
— a computer unit (28) for processing the signals representative of the measurements of total concentration of cations and of the conductivity, for deducing from them the concentration of free bases.

**Ansprüche**

1. Verfahren zur Bestimmung der Konzentration an freien Basen in mehrfach aufbereitetem Brauchwasser, d.h. an Basen oder Hydroxiden, welche in wässriger Lösung $OH^-$ Ionen abspalten, mit Ausnahme der zur Aufbereitung der wässrigen Lösung zugeführten Basen, wodurch die Schadstoffkonzentration in Form von Basen und Salzen geändert werden kann, bei welchem nach dem Durchrieseln des Wassers durch Ionenaustauschharze die Wasserleitfähigkeit gemessen wird, wobei die Kationen in der Lösung festgehalten und anstelle $H^+$-Ionen freigesetzt werden, und der Konzentrationswert der von diesen Salzen stammenden Kationen durch Anwendung der zwischen der basischen Leitfähigkeit und der aus den Salzen stammenden Kationenkonzentration bestehende lineare Beziehung aus dieser Messung bestimmt wird, dadurch gekennzeichnet, daß durch Assoziierung der mittels bekannten an sich für jeden evtl. anwesenden Kationentyp spezifischen Elektroden durchgeführten Messungen die gesamte Konzentration an aus den Salzen und Basen stammenden Kationen gemessen wird, und daß die Differenz der gesamten Kationenkonzentration und der aus den Salzen stammenden Kationenkonzentration errechnet wird, wobei das Ergebnis den Konzentrationswert der freien Basen im Wasser darstellt.

2. Verfahren zur Bestimmung der Konzentration nach Anspruch 1, bei dem Sekundärwasser eines Druckwasserkernreaktors, dessen Kondensator mittels Flußwasser gekühlt, dadurch gekennzeichnet, daß die gesamte Natrium- und Kalziumkonzentration im Wasser durch eine spezifische Elektrode (23) gemessen wird, daß die basische Wasserleitfähigkeit in einer mit einer Meßbrücke (25) verbundenen Leitfähigkeitszelle (22) gemessen wird, und daß die Konzentration an aus den Lösungssalzen stammenden Natrium und Kalzium aus der zwischen der Leitfähigkeit und der Natrium- und Kalziumkonzentration bestehende lineare Beziehung davon abgeleitet wird.

3. Verfahren zur Bestimmung der Konzentration an freien Basen nach Anspruch 1, bei dem Sekundärwasser eines Kernreaktors, dessen Kondensator mit Meerwasser gekühlt wird, dadurch gekennzeichnet, daß die gesamte Natriumkonzentration im Wasser durch eine potentiometrische Meßvorrichtung (29) mit einer spezifischen Elektrode gemessen wird, daß die basische Leitfähigkeit dieses Wassers gemessen wird, daß die Konzentration an aus den Lösungssalzen stammenden Natriumionen aus der zwischen der Leitfähigkeit und der Konzentration an Natriumsalzen bestehende lineare Beziehung davon abgeleitet wird.

4. Verfahren nach einem der Ansprüche 1, 2 und 3, dadurch gekennzeichnet, daß ein der gesamten Kationenschadstoffkonzentration im Wasser proportionales Signal generiert wird, daß ein zweites der Konzentration an aus den Salzen im Wasser stammenden Kationen proportionales Signal generiert wird, und daß ein für die Konzentration an freien Basen im Wasser typisches Signal durch Differenz zwischen den beiden Signalen bestimmt wird.

5. Verfahren zur Bestimmung der Konzentration an freien Basen nach einem der Ansprüche 1, 2 und 3, dadurch gekennzeichnet, daß die Gerade, welche die Konzentrationsschwankungen an aus den Salzen stammenden Kationen als Funktion der kationischen Leitfähigkeit darstellt, auf einem Diagramm aufgezeichnet wird, daß der Punkt, der als Abszisse die gemessene kationische Leitfähigkeit und als Ordinate die gesamte Konzentration an Schadstoffkationen in Wasser hat, auf diesem Diagramm aufgetragen wird und daß die Länge des zur Konzentrationsachse parallelen die Gerade mit dem Punkt verbindenden Segment bestimmt wird, wobei diese die Konzentrationsachse gleichziehende Länge die Konzentration an freien Basen ergibt.

6. Verfahren zur Bestimmung der Konzentration an freien Basen nach einem der Ansprüche 1, 2, 3, 4, und 5, dadurch gekennzeichnet, daß die gesamte Konzentration an Schadstoffkationen und die kationische Leitfähigkeit durch kontinuierliche Messungen bestimmt werden.

7. Meßvorrichtung zur Anwendung des Verfahrens nach den Ansprüchen 1, 2, 3, 4, 5 und 6, gekennzeichnet durch :
— eine Ionenaustauschkolonne (21),
— eine mit der Ionenaustauschkolonne in Reihe geschaltete Zelle (22) zur Messung der Leitfähigkeit,
— mindestens eine spezifische Elektrode (23) zur Messung der gesamten Kationenkonzentration,
— eine Recheneinheit (28) zur Verarbeitung der für die Messungen der gesamten Konzentration an Kationen und der Leitfähigkeit repräsentativen Signale um die Konzentration an freien Basen davon abzuleiten.

Fig1

Fig2

Fig 3

Fig 4

2